# EUROPEAN PATENT SPECIFICATION

(11) **EP 2 234 512 B1**
(45) Date of publication and mention of the grant of the patent: **12.04.2017**
(21) Application number: 09703879.8
(22) Date of filing: 23.01.2009
(51) Int. Cl.: A41D 13/06, A41D 13/08, A63B 71/12, A41D 13/05

(54) **JOINT PROTECTOR**
GELENKSCHUTZ
PROTECTION D'ARTICULATION

(30) Priority: 24.01.2008 SE 0800171
(43) Date of publication of application: 06.10.2010
(73) Proprietor: Otto Bock Scandinavia AB, 601 14 Norrköping (SE)
(72) Inventor: BOHLIN, Johan, S-614 90 Söderköping (SE)
(74) Representative: Ehrner & Delmar Patentbyrå AB
(86) International application number: PCT/SE2009/000025
(87) International publication number: WO 2009/093959

(56) References cited:
- WO-A1-2004/089132
- WO-A1-2006/073353
- DE-A1- 3 106 975
- JP-U- S54 134 569
- US-A- 3 703 171
- US-A- 4 064 874
- US-A1- 2001 007 929
- US-A1- 2003 083 605
- US-A1- 2007 060 857

## Description

### FIELD OF THE INVENTION

The invention concerns a protector according to the preamble of claim 1.

### BACKGROUND OF THE INVENTION

From WO2006/073353 and WO2004/089132 are previously known examples of tubular joint protectors from panel-shaped elastic material adapted for use on knee or an elbow. The protectors support and protect the joint they are applied on and allow movement at the same time as the joint maintains body heat so that strain and risks of injuries are limited.

Document US-A-3703171 describes an athletic knee supporter and protective device.

A problem with elastic protectors of this kind is the tendencies of sliding down, which risks occurring also during limited knee movements. The reason for this is that the movement of the knee result in alternating extension and shortening of the covering length of the protectors for the front and rear side of the leg.

The shape of the length portion above and under the knee is also altered during a knee movement because of volume variations of the muscle groups. This can further contribute strongly to downward displacement of the protector from the lower part of the thigh muscle. Also a slight corresponding effect occurs from the upper part of the lower leg muscle, which effect in that case gives the lower parts of the protector an upward movement. A total effect of these muscle influences easily result in that the previously known protector is easily pulled together towards the central portion of a knee.

### AIM AND THE MOST IMPORTANT FEATURES OF THE INVENTION

It is an aim to provide a protector as indicated initially for a knee or an elbow, wherein the problem of the background art is avoided or at least reduced.

These aims are achieved through the features of the independent claim.

Hereby is achieved that the material on the rear side of the protector is prevented from being displaced towards the hollow of the joint, wherein said disadvantages with displaced protector portions are avoided or at least essentially reduced. The strengthening of the protector also results in that the protector can be manufactured in materials that are more functionally adapted to certain purposes such as increased heat transfer from the knee area, which can be promoted through a thinner, more air-permeable textile.

The purpose of the support-rails is thus to stretch out and maintain the distance between the rear upper and lower edges of the protector, not to provide any supporting action against the region of the joint.

Through the invention is avoided to strive in the directions according to the development of the background art, which is to use rigid, relatively stable material in order to maintain shape, with resulting heat and comfort problems and abrasion etc.

It is preferred that the protector includes a textile material and that one textile pocket is arranged for receiving each said support-rail (rails). This textile material is then at least arranged in the rear region of the protector. Other portions of the protector can be of other kinds, for instance being shock-absorbing.

Each textile pocket is advantageously a channel being integrated in a textile portion.

It is preferred that it includes a plurality of support-rails being arranged in parallel to each other in order to ensure broadening of the effect with maintained comfort.

The protector most preferably includes a number of two to eight support-rails.

The material of the protector is advantageously at least partly elastic, which could not work in respect of a solution according to the background art. In particular, the material can at least partly be highly air-permeable in order to allow outward transport of heat and perspiration from the joint.

Each support-rail has preferably essentially the same dimension in its length extension as the protector in its length direction in order to achieve the highest degree of outward stretching of the protector.

The support-rail has suitably cross sectional dimensions of about 1 - 25 mm, and where a number of support-rails are used, each support-rail suitable has transverse dimensions of about 1 - 10 mm. Further advantages will be clear from the following detailed description.

### BRIEF DESCRIPTION OF DRAWINGS

The invention will now be described in more detail by way of embodiments and with reference to the annexed drawings, wherein:
Fig. 1 shows a protector according to the invention in a side view,
Fig. 2 shows the protector in Fig. 1 in a rear view, and
Fig. 3 shows a detail of the protector in Figs. 1 and 2, partly in section.

### DESCRIPTION OF EMBODIMENTS

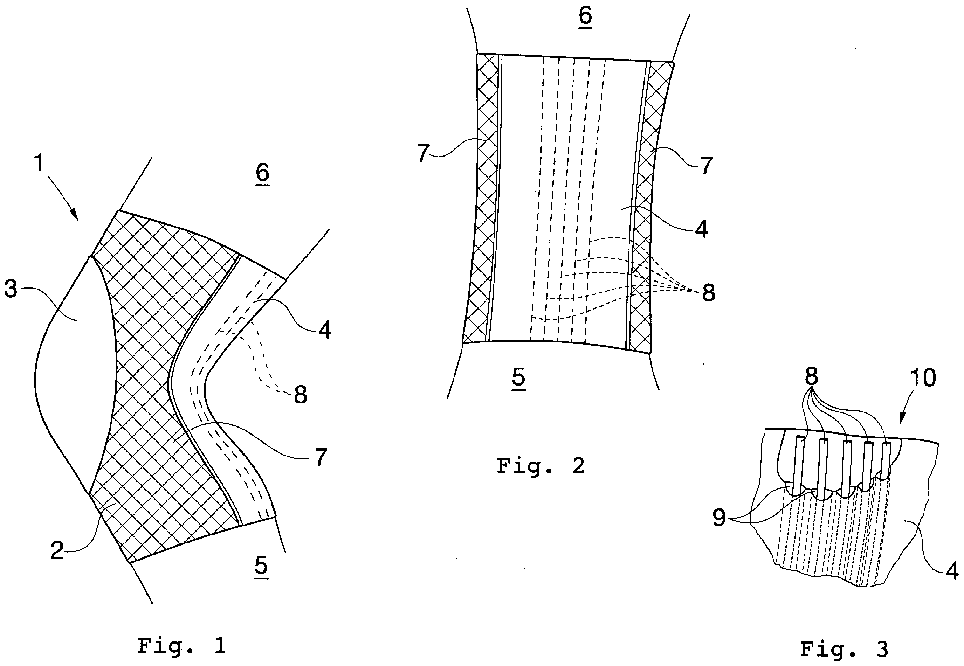

In Fig. 1 reference numeral 1 in general indicates a joint protector for a knee. The thigh of the carrier is indicated with 6 and lower leg with 5.

A joint protector 1 includes an essentially tubular structure 2, which in present case is a sports protector having a shock absorbing pad 3, which covers the front side of the knee in per se known manner. The tubular structure 2 further includes a rear piece 4 as well as a main piece 7 of a flexible and elastic textile material, which is highly air-permeable in order to allow good air exchange and thereby cooling of the user knee.

In Fig. 2 the protector 1 is shown from the rear, and with interrupted lines are indicated five parallel support-rails 8, which extend from the upper and lower edges of the join protector 1 and inside channels which are closed at the ends, inside the support structure.

The arrangement of the support-rails 8 is shown more clearly by the detail picture in Fig. 3. In this figure is shown an outside edge area of the rear piece 4, wherein five support-rails are shown extending from the partly cut away rear piece. Each support-rail 8 is arranged in a pocket or channel 9, which thus extends over the entire height of the rear piece 8 and is closed at the also cut away edge 10.

The channels 9 are separated from each other already through a manufacturing process of the textile portion which comprises the rear piece 4 such that the channels 9 are integral parts of this textile piece. On Fig. 3, the interrupted lines indicate how the support-rails 8 continue inside the channels 9 and the dotted lines indicate the limits sideways of the channels.

The invention can be modified within the scope of the following claims. The number of support-rails can be another that five and their shape can also be different. If one single support-rail is used, it can be strip-shaped and have essentially greater width than what is the case when a plurality of parallel support-rails is used.

What is important is, however, that the support-rails allow flexing of a joint protector which is essentially unrestrained by the rigidity of the support-rails. These are thus not a part of the supporting structure of the protector itself but have only the stretching function as is described above. Suitable material in the support-rails is a flexible thermoplastic material, which provides durable strength against bending.

Protectors according to the invention can also be used for other joints, such as elbows, wrists and ankles.

The protector has been described above as a joint protector with support-rail or support-rails positioned in the area of the hollow of the joint, and this embodiment is the essential and preferred for the invention. It is, however, not excluded that the protector can be used as protector for other body parts, such as for example a neck protector. In that case support-rails can be distributed in a suitable manner in front of and at the sides and even rearward on the protector, as seen when the protector is used, wherein these areas are inward articulating areas, what means that they are areas against which the body part articulates. Hereby is achieved inter alia the advantage that rigid and tight materials can be avoided in such protectors.

By the feature that the structure of the protector is given a configuration for adaption to a joint, is most simply intended that it is adapted as to dimensions. It can also be given a curved form from the beginning by the used material portions being cut and connected in a manner which is per se previously known.

The invention can be modified further within the scope of the annexed claims. The design of the protectors as well as the materials of different portions can be varied. The protector can thus include portions of non-elastic (but flexible) properties. It is not excluded that different seams or recesses can be brought on to different portions of the protector of for example aesthetic or other practical reasons.

## Claims

1. Protector (1) for an articulating body part, which protector at least partly includes a flexible material (7), and which includes an essentially tubular structure (2) with a length direction, essentially axially of the tubular structure (2), and a crosswise direction, which tubular structure (2) is given a configuration for adaption to the body part, **characterized in**
- **that** the protector includes at least one flexible support-rail (8) in a region (4) which is intended, in use of the protector, to be positioned in an inward articulating area of the body part, and
- **that** said at least one flexible support-rail (8) has a length extension essentially in parallel with the longitudinal direction of the protector, whereby the support-rail stretches out and maintains a distance between the rear upper and lower edges of the protector in said inward articulating area of the body part.

2. Protector according to claim 1, **characterized in that** it includes a textile material (4) and that for receiving said support-rail (rails), each one textile pocket (9) is arranged.

3. Protector according to claim 1 or 2, **characterized in that** each textile pocket (9) is comprised of a channel integrated in a textile portion (4).

4. Protector according to any one of the previous claims, **characterized in that** it includes a plurality of support-rails (8) being arranged generally in parallel with each other.

5. Protector according to any one of the previous claims, **characterized in that** it includes a number of two to eight support-rails (8).

6. Protector according to any one of the previous claims, **characterized in that** its material (7) is at least partly elastic.

7. Protector according to anyone of the previous claims, **characterized in that** its material (7) is at least partly highly air-permeable.

8. Protector according to any one of the previous claims, **characterized in that** each support-rail (8) has essentially the same dimension in its length extension as the protector (1) has in its longitudinal direction.

9. Protector according to any one of the previous claims, **characterized in that** each support-rail (8) has cross sectional dimensions of about 1 - 25 mm.

10. Protector according to any one of the previous claims, where a plurality of support-rails (8) are provided, **characterized in that** each support-rail (8) has cross sectional dimensions of about 1 - 10 mm.

11. Protector according to any one of the previous claims, **characterized in that** it includes a protective pad (3) on its front side.

12. Protector according to any one of the previous claims, wherein the articulating body part is a joint and the protector is a joint protector, **characterized in that** at said least one flexible support-rail (8) is positioned in the region of the hollow of the joint of the protector (2).

## Patentansprüche

1. Schutzmittel (1) für einen ein Gelenk bildenden Körperteil, wobei dieses Schutzmittel wenigstens teilweise ein flexibles Material (7) aufweist, und wobei es eine im Wesentlichen rohrförmige Struktur (2) mit einer Längenrichtung, die im Wesentlichen axial bezüglich der rohrförmigen Struktur (2) ist, und einer Querrichtung aufweist, wobei der rohrförmigen Struktur (2) eine Gestalt zur Anpassung an den Körperteil verliehen ist,
**dadurch gekennzeichnet,**
- **dass** das Schutzmittel wenigstens eine flexible Stützschiene (8) in einem Bereich (4) aufweist, welcher dazu vorgesehen ist, bei Gebrauch des Schutzmittels in einem sich einwärts beugenden Bereich des Körperteils positioniert zu sein, und
- **dass** die wenigstens eine flexible Stützschiene (8) eine Längenerstreckung aufweist, die im Wesentlichen parallel zur Längsrichtung des Schutzmittels ist,
wodurch sich die Stützschiene ausdehnt und einen Abstand zwischen dem hinteren oberen und hinteren unteren Rand des Schutzmittels in dem sich einwärts beugenden Bereich des Körperteils aufrechterhält.

2. Schutzmittel nach Anspruch 1, **dadurch gekennzeichnet, dass** es ein textiles Material (4) aufweist, und dass zum Aufnehmen der Stützschiene (Schienen) jeweils eine textile Tasche (9) angeordnet ist.

3. Schutzmittel nach Anspruch 1 oder 2,
**dadurch gekennzeichnet, dass** jede textile Tasche (9) aus einem Kanal besteht, der in einen textilen Abschnitt (4) integriert ist.

4. Schutzmittel nach einem der vorhergehenden Ansprüche,
**dadurch gekennzeichnet, dass** es mehrere Stützschienen (8) aufweist, die im Wesentlichen parallel zueinander angeordnet sind.

5. Schutzmittel nach einem der vorhergehenden Ansprüche,
**dadurch gekennzeichnet, dass** es eine Anzahl von zwei bis acht Stützschienen (8) aufweist.

6. Schutzmittel nach einem der vorhergehenden Ansprüche,
**dadurch gekennzeichnet, dass** sein Material (7) wenigstens teilweise elastisch ist.

7. Schutzmittel nach einem der vorhergehenden Ansprüche,
**dadurch gekennzeichnet, dass** sein Material (7) wenigstens teilweise hoch luftdurchlässig ist.

8. Schutzmittel nach einem der vorhergehenden Ansprüche,
**dadurch gekennzeichnet, dass** jede Stützschiene (8) im Wesentlichen dieselbe Abmessung in ihrer Längenerstreckung aufweist, wie sie das Schutzmittel (1) in seiner Längsrichtung aufweist.

9. Schutzmittel nach einem der vorhergehenden Ansprüche,
**dadurch gekennzeichnet, dass** jede Stützschiene (8) Querschnittsabmessungen von etwa 1 - 25 mm aufweist.

10. Schutzmittel nach einem der vorhergehenden Ansprüche, wobei mehrere Stützschienen (8) vorgesehen sind,
**dadurch gekennzeichnet, dass** jede Stützschiene (8) Querschnittsabmessungen von etwa 1 - 10 mm aufweist.

11. Schutzmittel nach einem der vorhergehenden Ansprüche,
**dadurch gekennzeichnet, dass** es ein Schutzpolster (3) auf seiner Vorderseite aufweist.

12. Schutzmittel nach einem der vorhergehenden Ansprüche,
wobei der ein Gelenk bildende Körperteil ein Gelenk ist und das Schutzmittel ein Gelenkschutz ist, **dadurch gekennzeichnet, dass** die wenigstens eine flexible Stützschiene (8) in dem Bereich der Aushöhlung des Gelenks des Schutzmittels (2) positioniert ist.

## Revendications

1. Dispositif de protection (1) pour une partie articulée du corps, le dispositif de protection comprenant au moins en partie un matériau flexible (7) et comprenant une structure (2) essentiellement tubulaire présentant une direction longitudinale disposée essentiellement axialement dans la structure tubulaire (2) et une direction transversale, la structure tubulaire (2) recevant une configuration permettant de l'adapter à la partie du corps, **caractérisé en ce que**
le dispositif de protection comprend au moins un rail flexible de support (8) dans une partie (4) qui est destinée à être placée dans une partie articulée intérieure de la partie du corps lorsque le dispositif de protection est utilisé et
**en ce que** ledit ou lesdits rails flexibles de support (8) présentent une extension en longueur essentiellement parallèle à la direction longitudinale du dispositif de protection,
le rail de support s'étendant et maintenant une distance entre le bord arrière supérieur et le bord arrière inférieur du dispositif de protection dans ladite partie articulée intérieure de la partie du corps.

2. Dispositif de protection selon la revendication 1, **caractérisé en ce qu'**il comprend un matériau textile (4) et **en ce qu'**une poche textile (9) est prévue pour recevoir ledit ou chacun desdits rails de support.

3. Dispositif de protection selon les revendications 1 ou 2, **caractérisé en ce que** chaque poche textile (9) est constituée d'un canal intégré dans une partie textile (4).

4. Dispositif de protection selon l'une quelconque des revendications précédentes, **caractérisé en ce qu'**il comprend plusieurs rails de support (8) agencés globalement en parallèle les uns aux autres.

5. Dispositif de protection selon l'une quelconque des revendications précédentes, **caractérisé en ce qu'**il comprend un nombre de deux à huit rails de support (8).

6. Dispositif de protection selon l'une quelconque des revendications précédentes, **caractérisé en ce que** son matériau (7) est au moins en partie élastique.

7. Dispositif de protection selon l'une quelconque des revendications précédentes, **caractérisé en ce que** son matériau (7) est au moins partiellement très perméable à l'air.

8. Dispositif de protection selon l'une quelconque des revendications précédentes, **caractérisé en ce que** chaque rail de support (8) présente essentiellement la même dimension dans son extension en longueur que celle du dispositif de protection (1) dans le sens de sa longueur.

9. Dispositif de protection selon l'une quelconque des revendications précédentes, **caractérisé en ce que** chaque rail de support (8) présente en section transversale des dimensions comprises entre 1 et 25 mm.

10. Dispositif de protection selon l'une quelconque des revendications précédentes, dans lequel plusieurs rails de support (8) sont prévus, **caractérisé en ce que** chaque rail de support (8) présente en section transversale des dimensions d'environ 1 à 10 mm.

11. Dispositif de protection selon l'une quelconque des revendications précédentes, **caractérisé en ce qu'**il comprend un tampon dispositif de protection (3) sur son côté frontal.

12. Dispositif de protection selon l'une quelconque des revendications précédentes, dans lequel la partie articulée du corps est une articulation et le dispositif de protection d'un dispositif de protection d'articulation, **caractérisé en ce que** ledit ou lesdits rails flexibles de support (8) sont disposés au niveau creux du joint du dispositif de protection (2).
